Europäisches Patentamt

European Patent Office

Office européen des brevets

(11) Veröffentlichungsnummer: **0 358 873 B1**

## EUROPÄISCHE PATENTSCHRIFT

(45) Veröffentlichungstag der Patentschrift: **08.06.94**

(51) Int. Cl.⁵: **A61M 1/34**, A61M 1/16

(21) Anmeldenummer: **89112416.6**

(22) Anmeldetag: **07.07.89**

(54) **Vorrichtung zur Bestimmung der Änderung des intravasalen Blutvolumens während der Blutfiltration in einer Blutreinigungseinrichtung.**

(30) Priorität: **13.08.88 DE 3827553**

(43) Veröffentlichungstag der Anmeldung:
**21.03.90 Patentblatt 90/12**

(45) Bekanntmachung des Hinweises auf die
Patenterteilung:
**08.06.94 Patentblatt 94/23**

(84) Benannte Vertragsstaaten:
**AT DE ES FR GB IT SE**

(56) Entgegenhaltungen:
**EP-A- 0 089 003**
**EP-A- 0 272 414**

**PATENT ABSTRACTS OF JAPAN, Band 10, Nr. 86 (P-443)[2143], 4. April 1986; & JP-A-60 222 748 (SUMITOMO BAKELITE K.K.) 07-11-1985**

**W. DRUKKER et al.: "Replacement of renal function by dialysis", 1983, pages 226-230, Martinus Nijhoff Publishers, Den Haag, NL * Seite 226: "Ultrasonic flow meters"; Seite 230 "Ultrasonic devices" ***

(73) Patentinhaber: **Fresenius AG**
**Gluckensteinweg 5**
**D-61350 Bad Homburg(DE)**

(72) Erfinder: **Polaschegg, Hans-Dietrich, Dr.**
**Grünwiesenweg 9**
**D-6370 Oberursel 4(DE)**

(74) Vertreter: **Dr. Fuchs, Dr. Luderschmidt Dr. Mehler, Dipl.-Ing Weiss Patentanwälte**
**Postfach 46 60**
**D-65036 Wiesbaden (DE)**

EP 0 358 873 B1

**Beschreibung**

Die Erfindung geht aus von einer Vorrichtung zum Messen der Änderung des intravasalen Blutvolumens nach dem Oberbegriff des Patentanspruchs 1, wie sie aus der EP-A-0 089 003 bekannt ist.

Bei Blutreinigungsverfahren, bei denen ein Flüssigkeitsaustausch oder ein Entzug von Flüssigkeit vorgesehen ist, ist es erforderlich, diesen Flüssigkeitsaustausch so zu steuern, daß ungewollte negative Effekte auf das Wohlbefinden des Patienten vermieden werden.

Verfahren, bei denen diese Notwendigkeit besteht, sind z.B. die Hämodialyse, die Hämofiltration und die Plasmafiltration.

Das Entfernen des Flüssigkeitsüberschusses aus dem Körper des Patienten erfordert ein sehr präzises Steuern der Flüssigkeitsbilanzierung, weshalb Dialyseverfahren auch nur mit flüssigkeitsbilanzierenden Vorrichtungen durchgeführt werden können. Trotz dieser präzisen Bilanzierung kommt es immer noch zu dialysetypischen Unannehmlichkeiten bei den Patienten, so zu Kopfschmerzen, Brechen und Muskelkrämpfen. Der Grund hierfür liegt vermutlich im zu raschen Entzug von Natriumionen aus dem Blut als Folge der Konzentrationsdifferenz von Natrium im Blut (extrakorporaler Kreislauf) und in der Dialysierflüssigkeit sowie im zu raschen Flüssigkeitsentzug.

Es ist bekannt, bei der Hämodialyse die sog. volumetrische Ultrafiltrationskontrolle durchzuführen. Stand der Technik der Ultrafiltrationskontrolle bei der Hämofiltration ist die Bilanzierung des Ultrafiltrats und der Substitutionslösung mit Hilfe von einer oder zwei Waagen (vgl. zum Beispiel die DE-OS 31 32 790). Diese bereits industriell eingesetzten Verfahren erlauben einen Flüssigkeitsentzug nach Vorgabe des Arztes bzw. des Bedieners, d.h. es wird nach Vorgabe über einen vorgegebenen Zeitraum eine bestimmte Menge Flüssigkeit dem Patienten entzogen. Es ist auch bekannt, ein sog. "Ultrafiltrationsprofil", d.h. einen zeitabhängigen Verlauf der Ultrafiltrationsrate vorzugeben. Durch die zeitliche Variation der Ultrafiltrationsrate soll die vorgegebene Ultrafiltrationsmenge möglichst schonend, d.h. insbesondere unter Vermeidung von Blutdruckabfällen, dem Patienten entzogen werden.

Zusätzlich kann durch Eingabe von physiologischen sowie von Behandlungsparametern in derartige Geräte zur Durchführung der Ultrafiltration die Änderung des intrazellulären und extrazellulären Volumens vorhergesagt werden. Da diese Änderungen das Wohlbefinden des Patienten deutlich beeinflussen, versucht der Bediener des Gerätes das Ultrafiltrationsprofil so zu gestalten, daß eine möglichst gleichmäßige Abnahme des Extrazellulärvolumens stattfindet. Obwohl dies zu einer Verbesserung des ansonsten unbefriedigenden Behandlungsergebnisses bei schwierig einzustellenden Patienten geführt hat, ist dieses Verfahren für die Routine zu aufwendig.

Aus der erwähnten EP-A-0 089 003 ist eine Blutreinigungeinrichtung bekannt, bei der im extrakorporalen Blutkreislauf eine Hämatokritmeßeinrichtung angeordnet ist, die an eine Kontroll- und Auswerteeinheit angeschlossen ist. Diese Hämatokritmeßeinrichtung beruht auf einer elektrischen Widerstandsmessung des Blutes während der Blutfiltration. Aus der Änderung der Widerstandswerte des Blutes wird die Änderung des Hämatokrits ermittelt und hieraus auf das intravasale Blutvolumen geschlossen. Derartige Widerstandsmessungen haben jedoch den Nachteil, daß die Meßwerte durch andere Einflußfaktoren, wie Flußrate, Erythrozytenorientierung usw. verfälscht werden.

Bei der DE-OS 36 40 089, die auf dem aus der EP-A-0 089 003 bekannten Prinzip aufbaut, wird aus den erhaltenen Werten einer Leitfähigkeitsrelativmeßanordnung und aus der Leitfähigkeit der frischen Dialysierflüssigkeit sowie dem Blutfluß und den Leistungsparametern des Dialysators die Plasmaleitfähigkeit, sowie die Änderung der Plasma- und Blutleitfähigkeit ermittelt. Anschließend wird der Hämatokrit während der Dialyse errechnet und aus dem Hämatokrit auf die Veränderung des intravasalen Blutvolumens geschlossen und in Abhängigkeit von der Veränderung des intravasalen Blutvolumens die Ultrafiltrationsrate bestimmt.

Eine weitere Vorrichtung zur Messung der Leitfähigkeit ist aus der EP-A-0029793 bekannt.

Andere Methoden zur Blutvolumenveränderungsmessung sind z.B. beschrieben in:
R.N. Greenwood, C. Aldridge, W.R. Cattell
Clinical Science (1984)66, 575-583:
"Serial blood water estimations and in-line blood viscometry: the continuous measurement of blood volume during dialysis procedures"
und U. Schallenberg, S. Stiller, H. Mann
Life Support Systems (1987) 5,
A New Method of Continuous Haemoglobinometric Measurement of Blood Volume During Haemodialysis".

Keines dieser bekannten Verfahren hat jedoch bisher zur industriellen Anwendung geführt, weil einerseits schwierig zu überwindende Meß- bzw. methodische Probleme und andererseits aufwendige bzw. zusätzliche Apparaturen, wie z.B. Leitfähigkeitsmeßeinrichtungen, erforderlich sind.

Aufgabe der Erfindung ist daher eine Vorrichtung, mit der diese Nachteile vermieden werden und mit der die Änderung des intravasalen Blutvolumens während der Filtration auf einfache Weise ermittelt werden kann.

Diese Aufgabe wird mit der Vorrichtung nach dem Anspruch 1 gelöst. Ausgestaltungen dieser Vorrichtung sind in den Unteransprüchen angegeben.

Die Vorrichtung nach dem Anspruch 1 weist mindestens einen Ultraschallsensor auf, der im extrakorporalen Blutkreislauf der Blutreinigungseinrichtung angeordnet ist, und eine Auswerteeinheit, die an diesen Ultraschallsensor angeschlossen ist. Die Auswerteeinheit ist derart ausgebildet, daß zu Beginn der Filtration ein erstes Ultraschallsignal gespeichert wird und während der Filtration die Veränderung der Ultraschallsignale ermittelt wird. Aus dieser Änderung wird die Veränderung des Hämatokrits ermittelt und daraus auf die Veränderung des intravasalen Blutvolumens geschlossen. Im extrakorporalen Blutkreislauf ist eine Temperaturmeßeinrichtung angeordnet, die an die Auswerteeinheit angeschlossen ist, welche zusätzlich derart ausgebildet ist, daß der Hämatokrit bezüglich Temperaturänderungen korrigiert wird.

Der Ultraschallsensor kann in den extrakorporalen Blutkreislauf einer Hämodialyseeinrichtung, einer Hämofiltrationseinrichtung oder einer Plasmafiltrationseinrichtung eingebaut werden.

In der Arbeit von E.L. Bradley und Jose Sacerio (" The velocity of ultrasound in human blood under varying physiologic parameters", Journal of Surgical Research 12, 290-297, 1972) werden Ultraschallmessungen am Humanblut beschrieben und die Zusammenhänge zwischen den Ultraschallsignalen und der Temperatur, des Hämatokrits und des Proteingehalts aufgezeigt. Aus dieser Arbeit geht auch hervor, daß im Megahertzbereich gearbeitet wird, wobei festgestellt wurde, daß die Schallgeschwindigkeit frequenzunabhängig ist.

In K. Kirk Shung ("Tissue Characterization with Ultrasound", Kap. 10, Seite 230) wird eine empirische Formel angegeben, die auf Arbeiten von Bradley und Sacerio beruht. Danach ist der Zusammenhang zwischen der Schallgeschwindigkeit c in m/sec, der Temperatur T in °C, des Hämatokrits H in % und des Proteingehalts w in g% wie folgt:

$$c = 1482,26 + 1,54\,T + 0,51\,H + 2,8\,w \qquad (1)$$

Setzt man die von K. Kirk Shung genannten Normalwerte ein, so erhält man

$$c = 1482,26 + 56,98 + 20,4 + 16,8 \qquad (2)$$

Daraus ersieht man, daß der Hämatokrit und der Proteingehalt in derselben Größenordnung eingehen.

Bei einem überwässerten Patienten, dessen Zustand durch Ultrafiltration korrigiert wird, verändern sich beide Werte proportional. Die Größenordnung dieser Veränderung liegt bei 10-20 %, was einer Änderung der Schallgeschwindigkeit von 3-6 m/sec entspricht, was wiederum der durch eine Temperaturänderung von 2°C hervorgerufenen Geschwindigkeitsänderung gleichkommt. Dies zeigt, daß eine Berücksichtigung der Temperatur erforderlich ist, weswegen im extrakorporalen Kreislauf auch die Temperaturmeßeinrichtung angeordnet und mit der Auswerteeinheit verbunden ist.

Mißt man daher die Temperatur T, so lassen sich aus der gemessenen Schallgeschwindigkeit Aussagen über den Hämatokrit machen.

Gemäß einer besonderen Ausführungsform der erfindungsgemäßen Vorrichtung ist im extrakorporalen Kreislauf mindestens noch eine Temperaturmeßeinrichtung angeordnet, die ebenfalls an die Auswerteeinheit angeschlossen ist, die derart ausgebildet ist, daß der Hämatokrit bezüglich der Temperatur korrigiert werden kann.

Wird auf diese Weise mit Hilfe des Ultraschallsensors vor Beginn der Behandlung der Hämatokrit $H_o$ und während der Behandlung der jeweilige Hämatokrit H gemessen, so läßt sich die Änderung des Blutvolumens dV wie folgt bestimmen:

$$\frac{dV}{V} = \frac{1-H_o}{H} \qquad (3)$$

Die wünschenswerte Auflösung von 1 % Änderung des Blutvolumens macht die Messung einer Relativveränderung der Ultraschallgeschwindigkeit von etwa 0,4 m/sec erforderlich. Dies sind etwa 0,03 % der Ultraschallgeschwindigkeit im Serum.

Hieraus lassen sich die Anforderungen an die Größe der Meßstrecke sowie an die Höhe der Meßfrequenz ableiten. Bei einer Laufstrecke von 1 cm beträgt die Laufzeit größenordnungsmäßig 6 $\mu$sec

und die gewünschte Auflösung 2 nsec. Diese Auflösung liegt noch im Bereich des mit konventionellen elektronischen Schaltungen Errreichbaren. Um diese Auflösung tatsächlich zu erreichen, ist eine Frequenz in der Größenordnung von MHz oder darüber erforderlich. Die Abschätzung der Wellenlänge ergibt für 1 MHz eine Wellenlänge von 1,5 mm.

Die Messung der Blutdichte kann mit einem Ultraschall-Sensor durchgeführt werden, der im arteriellen Teil des extrakorporalen Blutkreislaufes angeordnet ist.

Um die relative Änderung der Blutdichte während der Blutreinigung zu verfolgen, sind gemäß einer weiteren Ausführungsform im extrakorporalen Blutkreislauf zwei Ultraschallsensoren eingebaut, wovon der eine im arteriellen Teil des Blutkreislaufes und der zweite im venösen Teil des Blutkreislaufes angeordnet ist. Beide Ultraschallsensoren sind an die Auswerteeinheit angeschlossen, welche derart ausgelegt ist, daß eben diese zusätzliche relative Änderung der Ultraschallsignale während der Filtration erfaßt wird und hieraus die relative Veränderung des intravasalen Blutvolumens ermittelt werden kann.

Der Vorteil der beanspruchten Vorrichtung besteht weiterhin darin, daß die Ultraschallsensoren mit der Messung anderer Parameter, die ebenfalls mit Ultraschall gemessen werden, kombiniert werden kann, wodurch der apparative Aufwand in Grenzen gehalten werden kann.

So ist die Auswerteeinheit gemäß einer weiteren Ausführungsform mit einer Einrichtung zur Erkennung von Luft ausgestattet.

Weiterhin kann der Ultraschallsensor in einer Tropfkammer integriert sein und die Auswerteeinheit zum Nachweis des Flüssigkeitsniveaus ausgebildet sein. Vorzugsweise ist die Tropfkammer in diesem Fall mit einer Meßmarke versehen, die ultraschallreflektierend ist und die in definiertem, d.h. während der Messung unveränderlichem Abstand unterhalb des zu erwartenden Flüssigkeitsspiegels angeordnet ist. Das vom Sender ausgesandte Signal wird dann zweimal reflektiert, und zwar einmal an der Meßmarke und zum anderen an der Flüssigkeitsoberfläche. Aus dem ersten Signal läßt sich bei bekanntem Abstand die Schallgeschwindigkeit und aus dem Ergebnis sowie der Laufzeit des zweiten Signales das Niveau der Flüssigkeitsoberfläche bestimmen.

Des weiteren kann die Vorrichtung mit einer Dopplerdurchflußmeßeinrichtung kombiniert werden. Diese Kombinationsmöglichkeiten zeigen, daß eine Änderung des Blutvolumens ohne großen zusätzlichen technischen Aufwand bestimmt werden kann, wie es beim Stand der Technik erforderlich war.

Der Ultraschallsensor kann auf bekannte Weise mit einem Sender und einem gegenüberliegenden Empfänger ausgestattet sein, er kann aber auch unter Ausnutzung der Reflexion arbeiten. Zu diesem Zweck ist der Empfänger nicht gegenüberliegend angeordnet, sondern entsprechend dem Reflexionswinkel, der z.B. < 90° oder > 270° sein kann, angeordnet. Bei einem Reflexionswinkel von 180° ist der Empfänger mit dem Sender identisch.

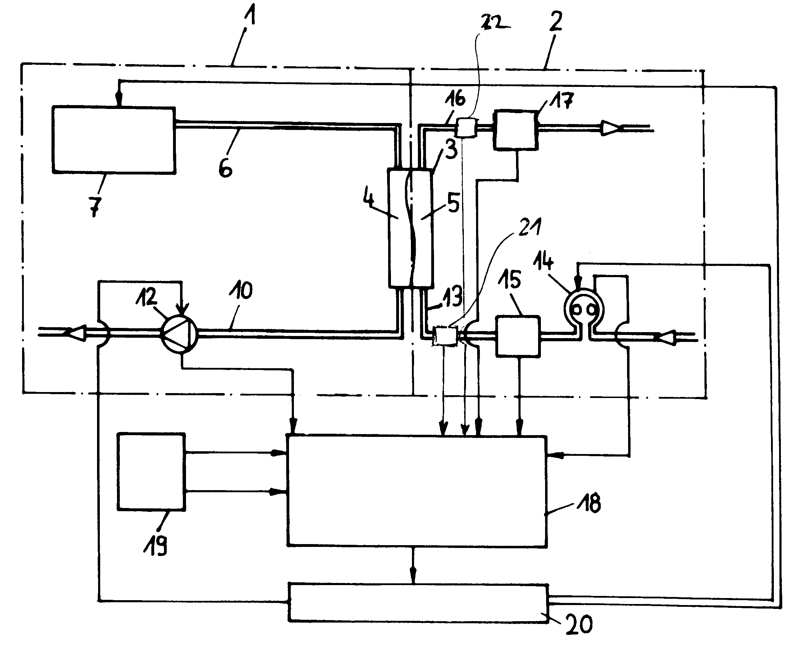

Nachfolgend wird ein Ausführungsbeispiel der Erfindung anhand der Zeichnung näher erläutert. In der Figur ist eine Hämodialyseeinrichtung dargestellt.

Die Hämodialysevorrichtung besteht im wesentlichen aus einem Dialysierflüssigkeitsteil 1 und einem extrakorporalen Blutkreislauf 2, zwischen denen sich ein Dialysator 3 mit einem Dialysierflüssigkeitskompartiment 4 und einem Blutkompartiment 5 befindet. Das Dialysierflüssigkeitskompartiment 4 ist stromauf des Dialysators 3 über eine Dialysierflüssigkeitsleitung 6 mit einer Dialysierflüssigkeitsquelle 7 verbunden. Stromab des Dialysators 3 ist dem Dialysierflüssigkeitskompartiment 4 eine weitere Leitung 10 nachgeordnet, die eine Dialysierflüssigkeitspumpe 12 aufweist.

Im extrakorporalen Blutkreislauf 2 sind in der Blutleitung 13 stromauf des Dialysators 3 eine erste Temperaturmeßeinrichtung 21 und ein erster Ultraschallsensor 15 angeordnet.

Mit dem Blutkompartiment 5 ist stromab des Dialysators 3 eine weitere Blutleitung 16 verbunden, die zusätzlich einen zweiten Ultraschallsensor 17 sowie eine weitere Temperaturmeßeinrichtung 22 aufweist. Letztere Temperaturmeßeinrichtung 22 ist erforderlich, um die bei der Dialyse auftretenden Temperaturverluste berücksichtigen zu können.

Die Dialysierflüssigkeitspumpe 12 und die Blutpumpe 14 sowie die Ultraschallsensoren 15 und 17 und die Temperaturmeßeinrichtungen 21 und 22 geben ihre Fördersignale bzw. ihre Meßwerte an eine Auswerteeinheit 18, in der die Bestimmung des Blutvolumens vorgenommen wird. Die Auswerteeinheit 18 steht weiterhin mit einer Eingabeeinheit 19 und einer Steuereinheit 20 in Verbindung, die ihre Signale an die Dialysierflüssigkeitsmischeinrichtung 7, die Dialysierflüssigkeitspumpe 12 und an die Blutpumpe 14 abgibt.

In der Dialysierflüssigkeitsmischeinrichtung 7 ist eine nicht näher dargestellte Konzentratpumpe und ein entsprechender Wasserzulauf angeordnet, wobei die Zusammensetzung der Dialysierflüssigkeit entsprechend dem Ausgangssignal der Steuereinheit 20 erfolgt.

Durch die während der Behandlung fortlaufende Bestimmung der Änderung des Blutvolumens mit Hilfe der Ultraschallsensoren 15 und 17 ist eine ständige Kontrolle der entzogenen Flüssigkeitsmenge möglich. Treten Abweichungen der vorgegebenen zu entziehenden Flüssigkeitsrate auf, die über die Eingabeeinheit 19 eingegeben wird, stellt die Auswerteeinheit 18 unter Berücksichtigung der Förderraten der Pumpen 14 und 12 fest, ob diese Förderraten geändert werden müssen oder die Dialysierflüssigkeitsmischeinrichtung anders angesteuert werden muß. Dies erfolgt durch entsprechende Signale, die an die Steuereinheit 20 gegeben werden.

**Patentansprüche**

1. Vorrichtung zum Messen der Änderung des intravasalen Blutvolumens durch Messen der Veränderung des Hämatokrits während der Blutfiltration in einer Blutreinigungseinrichtung mittels einer im extrakorporalen Blutkreislauf angeordneten Meßeinrichtung und einer daran angeschlossenen Auswerteeinheit (18), dadurch gekennzeichnet, daß diese Meßeinrichtung aus mindestens einem Ultraschallsensor (15) besteht, wobei diese Auswerteeinheit (18) in der Weise ausgebildet ist, daß zu Beginn der Blut-Filtration ein erstes Ultraschallsignal gespeichert wird und während der Filtration die Veränderung der Ultraschallsignale ermittelt wird, aus dieser Änderung die Veränderung des Hämatokrits ermittelt wird und daraus auf die Veränderung des intravasalen Blutvolumens geschlossen wird, und daß im extrakorporalen Blutkreislauf (2) eine Temperaturmeßeinrichtung (13) angeordnet ist, die an die Auswerteeinheit (18) angeschlossen ist, welche zusätzlich derart ausgebildet ist, daß der Hämatokrit bezüglich Temperaturänderungen korrigiert wird.

2. Vorrichtung nach Anspruch 1, dadurch gekennzeichnet, daß der Ultraschallsensor (15) im arteriellen Teil des extrakorporalen Blutkreislaufs (2) angeordnet ist.

3. Vorrichtung nach Anspruch 2, dadurch gekennzeichnet, daß ein zweiter Ultraschallsensor (17) im venösen Teil des extrakorporalen Blutkreislaufs (2) angeordnet ist, und daß beide Ultraschallsensoren (15, 17) an die Auswerteeinheit (18) angeschlossen sind, welche derart ausgebildet ist, daß zusätzlich die relative Veränderung der Ultraschallsignale während des Filtrierens erfaßt wird, aus dieser relativen Änderung die relative Änderung des Hämatokrits ermittelt wird und daraus auf die relative Veränderung des intravasalen Blutvolumens geschlossen wird.

4. Vorrichtung nach einem der Ansprüche 1 bis 3, dadurch gekennzeichnet, daß im extrakorporalen Blutkreislauf (2) eine zusätzliche Temperaturmeßeinrichtung (22) angeordnet ist.

5. Vorrichtung nach einem der Ansprüche 1 bis 4, dadurch gekennzeichnet, daß die Auswerteeinheit (18) mit einer Steuereinheit (20) zum Steuern der Filtration verbunden ist.

6. Vorrichtung nach einem der Ansprüche 1 bis 5, dadurch gekennzeichnet, daß die Auswerteeinheit (18) eine Einrichtung zum Erkennen von Luft aufweist.

7. Vorrichtung nach einem der Ansprüche 1 bis 6, dadurch gekennzeichnet, daß der Ultraschallsensor (15 oder 17) in eine Tropfkammer integriert ist und die Auswerteeinheit (18) zum Messen des Flüssigkeitsniveaus ausgebildet ist.

8. Vorrichtung nach einem der Ansprüche 1 bis 6, dadurch gekennzeichnet, daß die Auswerteeinheit (18) eine Dopplerdurchflußmeßeinrichtung aufweist.

**Claims**

1. Apparatus for measuring the change of the intravascular blood volume by measuring the change of the hematocrit during the blood filtration in a blood purification apparatus by means of a measuring device arranged in the extracorporeal blood circuit and an evaluating unit (18) connected thereto, characterized in that said measuring device consists of at least one ultrasonic sensor (15) and that said evaluating unit (18) is constructed in such a manner that at the start of the blood filtration a first ultrasonic signal is stored and during the filtration the change of the ultrasonic signals is determined, from said change the change of the hematocrit is determined and therefore the change of the intravascular blood volume deduced, and that in the extracorporeal blood circuit (2) a temperature measuring device (13) is arranged which is connected to the evaluating unit (18) which is additionally configured such that the hematocrit is corrected with respect to temperature changes.

**2.** Apparatus according to claim 1, characterized in that the ultrasonic sensor (15) is arranged in the arterial part of the extracorporeal blood circuit (2).

**3.** Apparatus according to claim 2, characterized in that a second ultrasonic sensor (17) is arranged in the venous part of the extracorporeal blood circuit (2) and that both the ultrasonic sensors (15, 17) are connected to the evaluating unit (18) which is constructed in such a manner that in addition the relative change of the ultrasonic signals during the filtering is detected, from said relative change the relative change of the hematocrit determined and therefrom the relative change of the intravascular blood volume deduced.

**4.** Apparatus according to any one of claims 1 to 3, characterized in that in the extracorporeal blood cicuit (2) an additional temperature measuring device (22) is arranged.

**5.** Apparatus according to any one of the claims 1 to 4, characterized in that the evaluating unit (18) is connected to a control unit (20) for controlling the filtration.

**6.** Apparatus according to any one of the claims 1 to 5, characterized in that the evaluating unit (18) comprises a means for detecting air.

**7.** Apparatus according to any one of the claims 1 to 6, characterized in that the ultrasonic sensor (15, or 17) is integrated into a drip chamber and the evaluating unit (18) is configured for measuring the liquid level.

**8.** Apparatus according to any one of claims 1 to 6, characterized in that the evaluating unit (18) comprises a Doppler flow measuring device.

**Revendications**

**1.** Dispositif pour la mesure du changement du volume de sang intravasculaire à l'aide des mesures du changement de l'hématocrite au cours de la filtration du sang dans une installation de purification du sang à l'aide d'un dispositif de mesure incorporé dans le circuit de sang extracorporel et d'une unité d'évaluation (18) reliée à celui-ci caractérisé en ce que ce dispositif de mesure se compose d'au moins un capteur d'ultrasons (15), ladite unité d'évaluation (18) étant conçue de telle façon qu'on mémorise un premier signal d'ultrasons au début de la filtration du sang et on détermine pendant la filtration les changements des signaux d'ultrasons, qu'on détermine à partir de ce changement le changement de l'hématocrite et qu'à partir de celui-ci, on peut tirer des conclusions sur le changement du volume de sang intravasculaire, et que dans le circuit de sang extracorporel (2) est installé un dispositif de mesure de températures (13), qui est relié à l'unité d'évaluation (18), qui est de plus conçue de telle façon que l'hématocrite est corrigée en fonction des changements de la température.

**2.** Dispositif selon la revendication 1, caractérisé en ce que le capteur d'ultrasons (15) est installé dans la partie artérielle du circuit de sang extracorporel (2).

**3.** Dispositif selon la revendication 2, caractérisé en ce qu'un deuxième capteur d'ultrasons (17) est installé dans la partie veineuse du circuit de sang extracorporel, et
en ce que les deux capteurs d'ultrasons (15, 17) sont reliés à l'unité d'évaluation (18), qui est conçue de telle façon qu'elle capte de plus les changements relatifs des signaux d'ultrasons au cours de la filtration et qu'elle détermine à partir de ce changement relatif, le changement relatif de l'hématocrite et qu'à partir de celui-ci, on peut tirer des conclusions sur le changement relatif du volume de sang intravasculaire.

**4.** Dispositif selon les revendications 1 à 3 caractérisé en ce que dans le circuit de sang extracorporel (2) est installé un dispositif de mesure de température (22) supplémentaire.

**5.** Dispositif selon l'une des revendications 1 à 4, caractérisé en ce que l'unité d'évaluation (18) est reliée à l'unité de commande (20) pour piloter la filtration.

6. Dispositif selon l'une des revendications 1 à 5, caractérisé en ce que l'unité d'évaluation (18) présente une installation de détection d'air.

7. Dispositif selon l'une des revendications 1 à 6, caractérisé en ce que le capteur d'ultrasons (15 ou 17) est intégré dans une chambre à brouillard et en ce que l'unité d'évaluation (18) est équipée pour mesurer le niveau de liquide.

8. Dispositif selon l'une des revendications 1 à 6 caractérisé en ce que l'unité d'évaluation (18) présente un dispositif de mesure de débit par effet Doppler.